## Europäisches Patentamt

## European Patent Office

## Office européen des brevets

(19)

(11) Numéro de publication: **0 175 607**
**B1**

(12) # FASCICULE DE BREVET EUROPÉEN

(45) Date de publication du fascicule du brevet:
14.10.87

(51) Int. Cl.⁴: **C 12 M 1/04, C 12 M 1/36**

(21) Numéro de dépôt: **85401658.1**

(22) Date de dépôt: **20.08.85**

(54) **Procédé et installation de culture de microorganismes.**

(30) Priorité: **31.08.84 FR 8413475**
**29.05.85 FR 8508027**

(43) Date de publication de la demande:
**26.03.86 Bulletin 86/13**

(45) Mention de la délivrance du brevet:
**14.10.87 Bulletin 87/42**

(84) Etats contractants désignés:
**AT BE CH DE FR GB IT LI LU NL SE**

(56) Documents cité:
**EP-A-0 041 702**
**EP-A-0 073 675**
**FR-A-710 646**
**FR-A-2 163 594**

(73) Titulaire: **L'AIR LIQUIDE, SOCIETE ANONYME POUR L'ETUDE ET L'EXPLOITATION DES PROCEDES GEORGES CLAUDE, 75, Quai d'Orsay, F-75321 Paris Cedex 07 (FR)**

(72) Inventeur: **Girardon, Philippe, 2, bis rue du Hazard, F-78000 Versailles (FR)**
Inventeur: **Brondeau, Pierre, 67, boulevard Lefèbvre, F-75015 Paris (FR)**
Inventeur: **Schvester, Pascal, 7, rue Mme de Montharzon La Ville aux Dames, F-37700 Saint-Pierre des Corps (FR)**
Inventeur: **Amen, Jean, 108, boulevard de la Reine, F-78000 Versailles (FR)**
Inventeur: **Crozel, Didier, 14, rue Edmond Roger, F-75015 Paris (FR)**
Inventeur: **Poillon, Dominique, 46, rue du Moulin, F-91430 Igny (FR)**

(74) Mandataire: **Leclercq, Maurice, L'AIR LIQUIDE SOCIETE ANONYME POUR L'ETUDE ET L'EXPLOITATION DES PROCEDES GEORGES CLAUDE 75, Quai d'Orsay, F-75321 Paris Cedex 07 (FR)**

LIBER, STOCKHOLM 1987

## Description

La présente invention concerne la culture de microorganismes. Les fermentations industrielles aércbies conduites dans des fermenteurs sont généralement aérées par de l'air, issu de compresseurs, avec des débits variant de 0,5 à 1 volume d'air par volume de milieu de culture et par minute. Les rendements de transfert de l'oxygène dans les réacteurs biologiques sont assez faibles. Ils sont généralement compris dans une fourchette de 3 à 40 % et, pour la majorité des cas, avoisinent 10 %. Certains cas particuliers présentent des rendements supérieurs pouvant aller jusqu'à 70 %.

Lorsque les cinétiques de croissance microbienne ou de production de métabolites sont limitées par une faible teneur en oxygène dissous dans le milieu de culture, un dopage de l'air avec de l'oxygène pur peut être effectué en régulant la teneur en oxygène dissous au-dessus de la concentration critique pendant une partie de la fermentation.

Lorsque la phase critique est longue, et lorsque la demande en oxygène des microorganismes est élevée, la quantité d'oxygène pur nécessaire au maintien d'une concentration en oxygène dissous au moins égale à la concetration critique est très importante. Compte tenu du faible rendement de transfert d'oxygène dans les réacteurs industriels, une partie importante de l'oxygène non utilisée est perdue dans les gaz effluents rendant ainsi le procédé peu économique. C'est pourquoi un recyclage des gaz effluents riches en oxygène constitue une solution d'amélioration de l'économie du procédé.

Ainsi, le document FR 2 163 594 résoud le problème de l'apport en oxygène au milieu de culture et de la régulation de sa température par injection de gaz à différents niveaux du milieu de culture; ce document évoque le recyclage des effluents, mais il ne pose ni le problème de l'enrichissement en $CO_2$ ni celui du rejet d'effluents riches en oxygène.

De même, le document EP 0 073 675 décrit le recyclage de tout ou partie des effluents dans un procédé continu de fermentation où les microorganismes sont fixés. Les effluents sont en outre enrichis en oxygène dans le circuit de recyclage. Cependant une partie des effluents, enrichie ou non, et donc une partie de l'oxygène, est rejetée et par conséquent perdue. L'épuration des effluents et le contrôle de l'oxygénation n'y sont pas précisément évoqués.

Certains procédés de recyclage de gaz déjà proposés mettent principalement l'accent sur la possibilité de contrôle de la concentration en gaz carbonique dissous par épuration du gaz carbonique, en éuilibre dans la phase gazeuse effluente, produit au cours de la réaction métabolique, l'accumulation progressive du gaz carbonique dissous devenant rapidement toxique pour la culture microbienne. Ces procédés d'épuration fonctionnent généralement sur le principe de fractionnement par liquéfaction, absorption ou adsorption du mélange gazeux. Ils ne sont toutefois pas compatibles avec une recirculation permanente de la totalité du débit de gaz effluent durant toute la phase de régulation de la teneur en oxygène dissous car nécessitant impérativement une interruption temporaire de fonctionnement, afin de permettre la régénération obligatoire du dispositif d'élinination du gaz carbonique. Une telle interruption ne permet donc qu'une recirculation partielle des gaz effluents. En fait, ces procédés répondent d'abord au problème d'inhibition et de toxicité causés par la présence du dioxide de carbone dans un milieu de fermentation. Ils constituent des dispositifs de contrôle et de régulation du gaz carbonique dissous, comme le procédé décrit au brevet français 710.646, plutôt qu'une solution d'utilisation optimale et continue de l'oxygène ayant servi à l'enrichissement des gaz d'aération des fermenteurs.

Enfin, le document EP 0 041 702 décrit le recyclage des effluents gazeux d'un milieu de culture et leur épuration du dioxyde de carbone par échange de chaleur entre les effluents et de l'oxygène et de l'azote liquide, le $CO_2$ alors solidifié étant évacué. Ce procédé consomme continuellement de l'oxygène pur et de l'azote cryogéniques, contrairement au procédé selon l'invention qui permet de réguler plus souplement la consommation d'oxygène pur.

Généralement la phase de régulation de l'oxygène dissous et de dopage à l'oxygène des gaz d'aération des fermenteurs peut excéder plusieurs heures. Le but de l'invention est de prévoir:

- des modalités optimales d'oxygénation combinant au cours de la fermentation l'oxygénation simple par de l'air, et l'oxygénation avec apport d'oxygène industriellement pur;

- une recirculation totale du volume du gaz d'aération;

- une épuration permanente et continue des gaz effluents de fementation jusqu'à des teneurs très basses en gaz carbonique (0 à 1 %);

- une regénération continue du système d'épuration des gaz par élimination du gaz carbonique retenu, et ce pour des durées pouvant excéder plusieurs heures.

Selon l'invention, on combine les mesures suivantes:

a) une phase initiale de la culture est assurée exclusivement par oxygénation avec de l'air atmosphérique, tandis qu'une phase subséquente à celle-ci est assurée exclusivement par circulation en recyclage d'une charge gazeuse constituée initiallement essentiellment par de l'air, auquel on adjoint, de façon régulée, de l'oxygène industriellement pur destiné à compenser substantiellement celui consommé par les microorganisnes et à maintenir le niveau d'oxygène dissous au minimum à sa valeur critique.

b) la commutation de la phase initiale à oxygénation exclusive par de l'air à la phase subséquente à oxygénation par adjonction exclusive d'oxygène industriellement pur s'effectue lorsque la teneur en oxygène disscus avoisine la valeur critique spécifique aux microorganismes cultivés.

De façon plus particulière:

c) l'épuration en dioxide de carbone de la charge gazeuse recyclée s'effectue par passage continu sur un adsorbant; par exemple du charbon actif.

Et dans un tel cas:

d) la pression d'épuration dans un adsorbeur et la pression de regénération dans un autre adsorbeur sont sensiblement voisines de la pression atmosphèrique.

Avantageusement, la durée des phases d'épuration, ou de regénération, des adsorbeurs est de l'ordre de la minute.

La commutation de la charge gazeuse de recyclage d'un adsorbeur en fin d'épuration vers un adsorbeur en fin de regénération s'effectue, pendant un bref laps de temps, en maintenant à l'atmosphère la sortie dudit adsorbeur en début d'épuration, de façon à éliminer la charge d'air qu'il contient.

L'invention vise en outre à appliquer ce procédé aux fermentations à haute densité cellulaire utilisant soit des systèmes d'immobilisation ou de floculation cellulaire, soit des systèmes à ultra-filtration ou micro-filtration éliminant les inhibiteurs de croissance, avec rétention et recyclage des microorganismes dans le fermenteur.

L'invention concerne également une installation de culture de microorganismes aérobies dans un substrat nutritif, du genre comprenant un fermenteur équipé d'une rampe de diffusion d'un gaz d'aération et d'une sonde de mesure de la teneur en oxygène dissous, d'une conduite d'alimentation en gaz d'oxygénation aboutissant à ladite rampe et d'une conduite d'évacuation des gaz effluents hors du fermenteur aboutissant à une boucle de recyclage incorporant un épurateur en eau et en gaz carbonique et un compresseur, qui est caractérisée en ce que:

a) la conduite d'évacuation des effluents comporte, en amont de la boucle de recyclage, une dérivation à vanne vers l'atmosphère;

b) la sonde de mesure de teneur en oxygène dissous agit sur un circuit de commutation incorporant d'une part des vannes de communication à un compresseur d'air du côté de la conduite d'alimentation en gaz d'oxygénation et à une vanne de mise à l'air en aval du fermenteur, et d'autre part au moins une vanne de raccordement à une source d'oxygène industriellement pur et une vanne de recyclage, ainsi que sur un mécanisme de commande de la mise en route ou de l'arrêt d'un compresseur de recyclage.

Selon une forme préférée de réalisation, l'installation comporte un jeu de deux adsorbeurs dont les entrées et les sorties sont reliées par des vannes au circuit de recyclage d'une part, et d'autre part par d'autres vannes à l'atmosphère et à un circulateur; et de préférence l'adsorbeur est un charbon actif. Avantageusement un moyen de commutation périodique de deux adsorbeurs en vue d'assurer successivement une phase d'épuration du gaz effluent pour l'un et une phase de regénération par l'air pour l'autre et vice-versa. Selon l'invention le fermenteur est associé à un dispositif d'ultrafiltration ou de microfiltration par des conduites de soutirage et de recyclage du milieu de culture aboutissant aux extrémités d'un compartiment amont d'une cellule d'ultrafiltration ou de microfiltration, dont un compartiment aval est raccordé à une conduite de décharge à vanne régulée.

Les caractéristiques et avantages de l'invention ressortiront d'ailleurs de la description qui suit à titre d'exemple en référence à la figure unique qui représente schématiquement une installation conforme à l'invention.

Un fermenteur 1 est équipé d'une rampe 3 d'injection d'un gaz d'aération véhiculé par une conduite 4 provenant d'une capacité-tampon 5. Le fermenteur 1 présente en partie haute une conduite d'évacuation 6 aboutissant d'une part à une vanne de mise à l'atmosphère 7 d'autre part à une vanne de recyclage 8 d'un circuit de recyclage 9 incorporant un échangeur-condenseur d'eau 10, une batterie de deux adsorbeurs 11, 12, avant d'aboutir à l'entrée d'un compresseur spécial oxygène 15 dont la sortie est raccordée par une conduite 16 à la capacité tampon 5.

En outre, la capacité-tampon 5 est raccordée par une conduite 20 à vanne 46 à un compresseur d'air 22 d'une part, par une conduite 26 à vanne 27 et via une vanne d'arrêt 28 à une source d'oxygène industriellement pur 29.

Le fementeur 1 est équipé d'une sonde de mesure d'oxygène dissous 30 qui commande, par l'intermédiaire d'un régulateur 31 et d'un comparateur 40 l'ouverture ou la fermeture des vannes 46 et 27 et cela de la façon suivante: lorsque la teneur en oxygène dissous est élevée, le régulateur 31 commande la fermeture de la vanne 27 et le comparateur 40, par l'intermédiaire du transmetteur 42, l'ouverture de la vanne 46. Lorsque la teneur en oxygène dissous descend à une valeur voisine du seuil critique, le régulateur 31 provoque l'ouverture de la vanne 27 et le comparateur 40, via le transmetteur 42, la fermeture de la vanne 46. La sonde 30 agit également par l'intermédiaire du comparateur 40 sur un organe de commande 41 du fonctionnement du compresseur 15 d'une part, et d'autre part, via le transmetteur 42 d'une part sur les vannes 7, 8 (pour ouvrir la vanne 7 et fermer la vanne 8 lorsque la teneur en oxygène dissous est élevée, pour fermer la vanne 7 et ouvrir la vanne 8 lorsque la teneur en oxygène dissous descend au voisinage de son seuil critique), sur un circulateur de régénération 43 pour les adsorbeurs 11 et 12.

Le transmetreur 42 asservi à la sonde 30 contrôle également via 50, un organe temporisé 51 commandant d'une part des vannes de sortie 52, 53 à la sortie des adsorbeurs 11 et 12, d'autre part des vannes d'entrée 54, 55 à l'entrée des adsorbeurs 11 et 12. Ce même organe temporisé commande également des vannes 56 et 57 respectivement branchées entre les sorties des adsorbeurs 11 et 12 et une conduite de mise à l'air 58 d'une part, d'autre part les vannes 59 et 60 reliant respectivement les entrées des adsorbeurs 11 et 12 au circulateur d'aspiration 43 vers l'atmosphère.

Le fonctionnement de l'installation qui vient d'être décrit est le suivant: on place dans le fermenteur 1 un substrat nutritif 70 que l'on ensemence avec le microorganisme de culture. La phase initiale de culture, pendant laquelle la teneur en oxygène dissous mesurée par la sonde 30 est relativement élevée, est assurée par une circulation d'air provenant du compresseur 22 et introduit dans la capacité 5 (vannes 21 et 46 ouvertes) puis dirigé par la conduite 4 vers la rampe d'injection 3. L'effluent gazeux sous-oxygéné et chargé en dioxide de carbone et vapeur d'eau évacué par la conduite 6 est mis à l'atmosphère par la vanne 7 en position d'ouverture, tandis que les vannes de recyclage 8 et d'alimentation en oxygène pur 27 restent fermées.

Cette phase initiale de culture de microorganismes se poursuit avec développement exponentiel de ceux-ci. Après un laps de temps, la teneur en oxygène dissous descend au voisinage immédiat d'un seuil critique et, à ce moment, la sonde 30 provoque la succession des opérations suivantes: la vanne 46 se ferme, tandis que la vanne 27 s'ouvre; la vanne 7 se ferme, tandis que la vanne 8 s'ouvre; l'organe de commande 41 met en route le compresseur 15, tandis que le transmetteur 42 ferme la vanne 46 et provoque la mise en service de l'organe temporisé 51.

A la suite de cette commutation, le gaz, qui est injecté dans la rampe 3 est constitué d'une charge gazeuse formée initialement d'air, occupant les circuits et le ciel du fermenteur, c'est-à-dire ayant une importante proportion d'azote qui est donc recyclée en circuit fermé via les conduites 6, 9, 16, 5 et 4.

La sonde 30 provoque l'admission d'oxygène dans la capacité-tampon 5 en provenance de la source 29, de façon à maintenir la valeur de l'oxygène dissous légèrement au-dessus du seuil critique, tandis que le gaz effluent issu est amené d'abord par la conduite 9 vers le condenseur d'eau 10, où la vapeur d'eau contenue dans le gaz recyclé est condensée, après quoi ce même gaz effluent est amené dans l'un ou l'autre des deux adsorbeurs 11 (ou 12) en fonctionnement en phase d'épuration, tandis que l'autre adsorbeur 12, (11) respectivement est en phase de regénération; la charge gazeuse ainsi traitée est débarrassée de son dioxyde de carbone, qui est piégé par le charbon actif de l'adsorbeur 11 (12) et le gaz ainsi épuré est transféré de nouveau à l'entrée du compresseur 15 et de là vers la capacité-tampon 5. De façon périodique et avec une période de l'ordre de la minute, l'organe temporisé 51 provoque la commutation de toutes les vannes (52, 53) (54, 55) (56, 57) (59, 60), ce qui fait permuter les phases d'épuration et de regénération des adsorbeurs 11 et 12.

Ainsi, comme on le constate, la phase de regénération d'un adsorbeur 11 ou 12 du début est assurée par simple passage à contre-courant d'air prélevé en 58 et aspiré par le circulateur 43 et rejeté à l'air. On précise que lors d'une permutation, l'adsorbeur 11 (12) qui commence sa phase d'épuration du gaz effluent n'est pas isolé immédiatement de l'air extérieur par la fermeture immédiate de la vanne 56 (ou 57), mais que cette vanne 56 (ou 57) voit sa fermeture différée quelque peu, de façon à assurer, pendant ce laps de temps, la mise à l'atmosphère de l'air contenant une forte proportion d'azote dans l'adsorbeur 11 (ou 12) en fin de regénération, évitant ainsi un accroissement inadmissible de la charge d'azote circulant en cours de recyclage.

A titre d'exemple de réalisation, une unité d'épuration et de recirculation des gaz de fermentation et installée sur un fermenteur servant à la production d'un antibiotique aéré par un gaz circulant avec un débit de 3000 Nm3/h qui, en phase d'enrichissement en oxygène, a la composition suivante: 50 % O2, 50 % N2. Ce gaz traverse le fermenteur où une partie de l'oxygène est consommée par le métabolisme des microorganismes et ressort de ce même fermenteur en ayant les compositions suivantes: 45 % O2, 50 % N2, 5 % CO2. La recirculation de ce gaz implique au préalable une étape de déshydratation et une étape d'élimination du CO2. Le gaz effluent ayant un débit de 3000 Nm3/h est dirigé vers la batterie d'adsorbeurs et passe sur une colonne remplie avec 2,5 à 3 m3 de charbon actif dont les caractéristiques sont voisines du "CEA AC 40". Le gaz est alors épuré et sa teneur en CO2 en sortie d'adsorbeur est abaissée à 1 % maximum. Par passage au travers d'un compresseur, le gaz épuré est recomprimé à 4 - 5 bars et réenrichi en oxygène pur avant réinjection à la base du fermenteur. Le rendement en oxygène recyclé de cet épurateur est proche de 90 %.

Comme autre exemple de réalisation, on signale une unité d'épuration et de recirculation des gaz de fermentation installée sur un fermenteur servant à la production de levures. Ce fermenteur est oxygéné par un gaz ayant un débit de 3000 Nm3/h qui, en phase d'enrichissement en oxygène a la composition suivante: 50 % O2, 50 % N2. A la sortie du fermenteur, ce même gaz a la composition suivante: 30 % O2, 20 % CO2, 50 % N2. Ce gaz effluent ayant un débit de 3000 Nm3/h est dirigé vers la batterie d'adsorbeurs et passe sur une colonne remplie avec 5 m3 de charbon actif dont les caractéristiques sont voisines du "CECA AC 40". Le gaz est alors épuré et sa teneur en CO2 en sortie d'adsorbeur est abaissé à 3 %. Le rendement en oxygène recyclé de cet épurateur est proche de 90 %.

A titre de troisième exemple de réalisation, une unité d'épuration et de recirculation des gaz de fermentation est installée sur un fermenteur servant à la production d'un acide aminé. Ce fermenteur est oxygéné par un gaz ayant un débit de 3000 Nm3/h qui, en phase d'enrichissement en oxygène a la composition suivante: 40 % O2, 60 % N2; à la sortie du fermenteur, ce même gaz a la composition suivante: 20 % O2, 20 % CO2, 60 % N2. Ce gaz effluent ayant un débit de 3000 Nm3/h est dirigé vers la batterie d'adsorbeurs et passe sur une colonne remplie avec 2,5 à 3 m3 de charbon actif. Le gaz est alors épuré et sa teneur en CO2 en sortie d'adsorbeur est abaissée à 1 %. Le rendement en oxygène recyclé de cet épurateur est proche de 90 %.

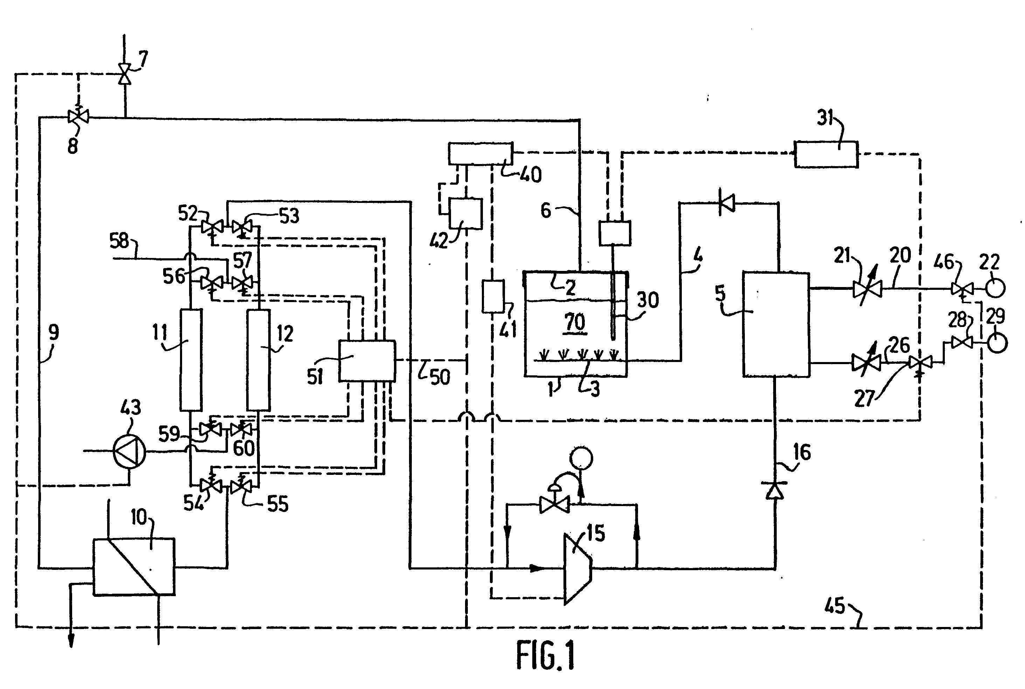

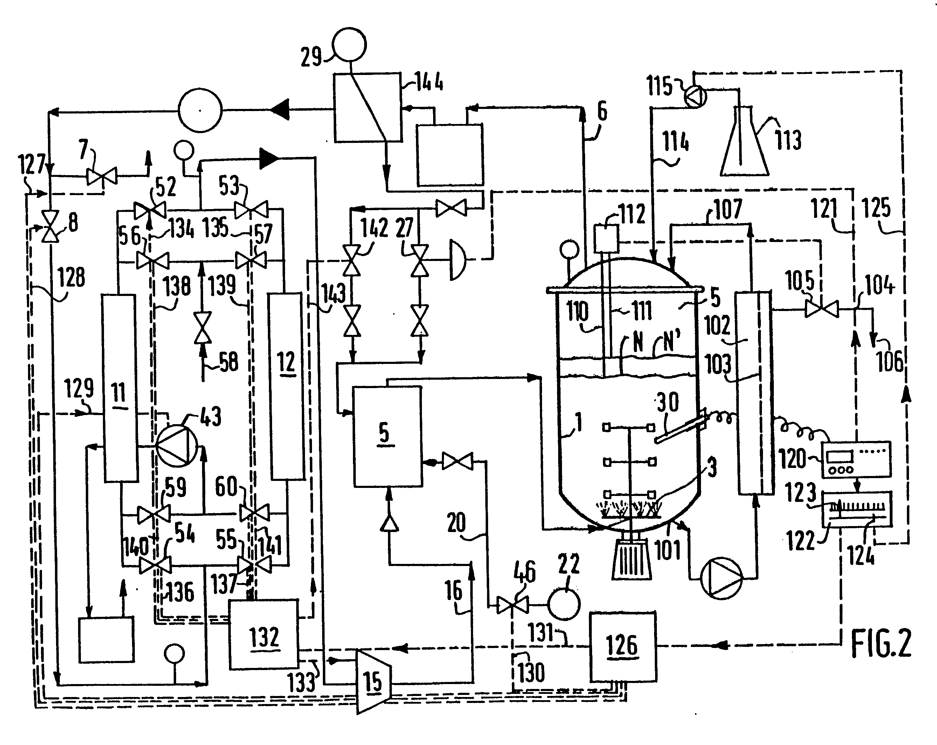

Selon la variante de la figure 2, on retrouve certains éléments de la figure 1, désignés par les mêmes chiffres de référence, notamment le fermenteur 1, avec la rampe injectrice 3, la sonde de mesure de la teneur en oxygène dissous 30, la capacité-tampon 5 alimenté soit en air comprimé 22 via la conduite 20 et l'électrovanne 46, soit en oxygène industriel provenant d'une source 29 via l'électrovanne 27, la conduite de soutirage d'effluent gazeux 6 vers soit une électrovanne de mise à l'air 7, soit une électrovanne de recyclage 8 vers des adsorbeurs 11 et 12 de piégeage du gaz carbonique, dont la sortie est raccordé par une conduite 16 à compresseur 15 au réservoir-tampon 5.

A la différence de la réalisation précédente, on a affaire ici à un fermenteur 1 avec dispositif d'élimination des inhibiteurs métaboliques par circulation du milieu de culture prélevé en 101 et dirigé vers l'entrée d'un dispositif 102 à membrane d'ultrafiltration 103 ou de microfiltration éliminant par perméation les inhibiteurs et une partie minimale du substrat, sans laisser passer les microorganismes. Une conduite 104 à électrovanne 105 dirige le perméat vers une évacuation 106, tandis qu'un conduit 107 recycle le milieu de culture épuré des inhibiteurs dans le fermenteur 1. Pour assurer un niveau sensiblement constant au moins dans une phase de culture mettant en oeuvre une ultrafiltration, le fermenteur est équipé d'une sonde double de niveau 110 (valeur basse) et 111 (valeur haute) commandant via un régulateur 112 l'électrovanne de perméat 105 de façon à limiter le débit de perméation si le niveau baisse en dessous de N et au contraire de l'accroître si le niveau tend à s'élever au dessus de N'. Ici, en outre, est prévue une alimentation en substrat nutritif à partir d'une réserve 113 et une conduite 114 à pompe 115. L'apport de substrat est régulé en fonction des exigences nutritives de la souche microbienne cultivée de façon à avoir en permanence une utilisation optimisée dudit substrat. Afin de minimaliser à l'extrême les pertes de substrats au travers du dispositif de perméation, l'apport de ceux-ci se fait sous forme diluée. De cette manière, le substrat est consommé avant d'avoir la possibilité de traverser la membrane de perméation du fait du faible gradient de concentration régnant de part et d'autre de la membrane. La mise en route de l'apport de substrat est automatique et peut être asservie au signal donné par différents capteurs présents sur le réacteur comme le pH, le rH, ou l'oxygène dissous. D'une façon plus particulière, on décrit l'asservissement à la concentration en oxygène dissous. Dans ce cas, la sonde d'oxygène dissous 30 commande via un régulateur 120 non seulement l'électrovanne d'alimentation en oxygène 27 via 121, mais également un multirégulateur 122 à affichage bas 123 et haut 124 commandant via 125 l'ouverture ou la fermenture de la pompe d'apport du substrat nutritif 115, l'affichage bas 123 commandant via un programmeur pricipal 126 l'électrovanne de mise à l'air 7 (via 127), l'électrovanne de recyclage 8 (via 128), le ventilateur de régénération 11 (via 129), l'électrovanne d'air comprimé 46 (via 130), selon le mode opératoire décrit précédemment.

Le programmeur principal 126 commande également via 131 un second programmeur 132 qui assure:
- d'une part la commande du fonctionnement du compresseur de recyclage 15 (via 133);
- d'autre part la commande des électrovannes 52, 53, 54, 55, 56, 57, 59, 60 du dispositif adsorbeur 11, 12 (via 134, 135, 136, 137, 138, 139, 140 et 141).

Le fonctionnement du dispositif d'alimentation du fermenteur en gaz oxygéné est du type décrit précédemment, mais ici la sonde de mesure d'oxygène dissous 30 commande en outre via 122, 124, 135, la mise en route de la pompe 115 d'alimentation en substrat, de façon à maintenir l'activité métabolique dans le réacteur provoquant ainsi une chute de la concentration en oxygène dissous en dessous de la valeur maximale affichée en 124.

La source d'oxygène industriel 29 est soit fournie par un dispositif adsorbeur enrichissant l'air en oxygène, soit par un dispositif de perméation, soit par un réservoir d'oxygène liquide. Dans ce dernier cas, on utilise une partie de l'énergie frigorifique de réchauffement de cet oxygène pour refroidir l'effluent gazeux issu du fermenteur 5 dans un échangeur 144.

Dans le cas où les caractéristiques du milieu de culture ne permettent pas une mesure correcte de l'oxygène dissous au moyen d'un capteur directement incorporé dans ce milieu, il convient de piloter l'apport de substrat à partir de mesures indirectes faites sur les fluides effluents provenant du fermenteur.

Le procédé combine ainsi un dispositif d'aération avec utilisation optimale de l'oxygène avec un dispositif assurant une productivité maximale pour lequel l'apport et l'utilisation du substrat ont été optimalisés et adoptés aux exigences métaboliques du microorganisme cultivé.

## Revendications

1. Procédé de culture de microorganismes en aérobiose dans un milieu nutritif liquide soumis à une oxygénation maintenant la teneur en oxygène dissous dans ledit milieu au-dessus d'un seuil critique caractérisé par les étapes suivantes:

a) une phase initiale de la culture est assurée exclusivement par aération avec de l'air atmosphèrique, tandis qu'une phase subséquente à celle-ci est assurée exclusivement par circulation en recyclage d'une charge gazeuse constituée initialement essentiellement par de l'air, auquel on adjoint, de façon régulée, de l'oxygène industriellement pur destiné à compenser substantiellement celui consommé.

b) la commutation de la phase initiale à oxygénation exclusive par de l'air.à la phase subséquente à oxygénation par adjonction exclusive d'oxygène industriellement pur s'effectue lorsque la teneur en oxygène dissous avoisine la valeur critique spécifique aux microorganismes cultivés.

2. Procédé de culture selon la revendication 1, caractérisé en ce que l'épuration en dioxide de carbone de la charge gazeuse recyclée s'effectue par passage sur un adsorbant constitué par du charbon actif.

3. Procédé de culture selon la revendication 2, caractérisé en ce que la pression d'épuration dans un adsorbeur et la pression de regénération dans un autre adsorbeur sont sensiblement voisines de la pression atmosphèrique.

4. Procédé selon la revendication 3, caractérisé en ce que la durée des phases d'épuration, ou de regénération, des adsorbeurs est de l'ordre de la minute.

5. Procédé de culture selon la revendication 4, caractérisé en ce que la commutation de la charge gazeuse de recyclage d'un adsorbeur en fin d'épuration vers un adsorbeur en fin de regénération s'effectue, pendant un bref laps de temps, en maintenant à l'atmosphère la sortie dudit adsorbeur en début d'épuration, de façon à éliminer la charge d'air qu'il contient.

6. Procédé de culture de microorganismes selon la revendication 1, caractérisé en ce qu'il est appliqué plus particulièrement aux fermentations à haute densité cellulaire notament aux fermentations à ultrafiltration ou microfiltration permettant d'éliminer des inhibiteurs de croissance, avec rétention et recyclage dans le fermenteur des microorganismes.

7. Procédé selon la revendication 6, caractérisé en ce que le substrat est apporté sous forme diluée en fonction des besoins métaboliques des microorganismes et intégralement consommé par ces microorganismes.

8. Installation de culture de microorganismes aerobies dans un substrat nutritif, du genre comprenant un fermenteur à couvercle étanche équipé d'une rampe inférieure de diffusion d'un gaz d'oxygénation et d'une sonde de mesure de la teneur en oxygène dissous, d'une conduite d'alimentation en gaz d'oxygénation aboutissant à ladite rampe et d'une boucle de recyclage incorporant un adsorbeur et un compresseur, caractérisé par les mesures suivantes:

a) la conduite d'évacuation des effluents comporte, en amont de la boucle de recyclage, une dérivation à vanne vers l'atmosphère;

b) l'installation comporte un jeu de deux adsorbeurs dont les entrées et les sorties sont reliées par des vannes au circuit de recyclage d'une part, et d'autre part par d'autres vannes à l'atmosphère et à un circulateur;

c) l'adsorbant est un charbon actif;

d) la sonde de mesure de teneur en oxygène dissous agit sur un circuit de commutation incorporant d'une part des vannes de communication à un compresseur d'air du côté de la conduite d'alimentation et à une vanne de mise à l'air en aval du fermenteur, et d'autre part au moins une vanne de raccordement à une source d'oxygène industriellement pur et une vanne de recyclage, ainsi que sur un mécanisme de commande de la mise en route ou de l'arrêt d'un compresseur de recyclage.

9. Installation de culture de microorganismes selon la revendication 8, caractérisée en ce qu'elle comporte un jeu de deux adsorbeurs dont les entrées et les sorties sont reliées par des vannes au circuit de recyclage d'une part, et d'autre part par d'autres vannes à l'atmosphère et à un circulateur.

10. Installation de culture de microorganismes selon la revendication 9, caractérisée en ce qu'elle comporte un moyen de commutation périodique de deux adsorbeurs en vue d'assurer successivement une phase d'épuration du gaz effluent pour l'un et une phase de regénération par l'air pour l'autre et vice-versa.

11. Installation de culture de microorganismes selon la revendication 8, caractérisée en ce que le fermenteur est associé à un dispositif d'ultrafiltration ou de microfiltration par des conduites de soutirage et de recyclage du milieu de culture aboutissant aux extrémités d'un compartiment amont d'une cellule d'ultrafiltration ou de microfiltration, dont un compartiment aval est raccordé à une conduite de décharge à vanne régulée.

12. Installation de culture de microorganismes selon la revendication 11, caractérisée en ce que la vanne de réglage de débit de filtrat est asservie à une sonde de niveau du milieu de culture dans le fermenteur.

13. Installation selon la revendication 11, caractérisée en ce que le fermenteur est raccordé à une conduite d'alimentation en substrat nutritif incorporant une pompe de débit asservie au signal d'un capteur installé sur l'unité de fermentation ou sur les canalisations des fluides effluents provenant du fermenteur.

14. Installation selon la revendication 10, caractérisée en ce que le capteur est une sonde à oxygène dissous ou une sonde pH ou une sonde de mesure de concentration de substrat.

## Patentansprüche

1. Verfahren zum Züchten von Mikroorganismen unter aeroben Bedingungen in einem flüssigen Nährmilieu, welches einer Sauerstoffanreicherung unterworfen wird, wobei der Gehalt an gelöstem Sauerstoff in diesem Milieu über einer kritischen Schwelle gehalten wird, gekennzeichnet durch die folgenden Stufen:

a) eine Anfangsphase der Kultur wird ausschließlich durch Belüftung mit atmosphärischer Luft sichergestellt, während eine dieser folgende Phase ausschließlich durch Zirkulation unter Rezyklieren einer gasförmigen Charge sichergestellt wird, die anfänglich im wesentlichen aus Luft besteht, der man in geregelter Weise industriell reinen Sauerstoff zuführt, wobei der Sauerstoff dazu bestimmt ist, im wesentlichen den verbrauchten Sauerstoff zu kompensieren; wobei

b) die Vertauschung der Anfangsphase mit Sauerstoffanreicherungausschließlich durch Luft mit der nachfolgenden Phase mit Sauerstoffanreicherung durch Zuführung ausschließlich von industriell reinem Sauerstoff erfolgt, wenn der Gehalt an gelöstem Sauerstoff in die Nähe des kritischen Wertes kommt, welcher für gezüchtete Mikroorganismen spezifisch ist.

2. Züchtungsverfahren nach Anspruch 1, dadurch gekennzeichnet, daß die Reinigung der rezyklierten, gasförmigen Charge von Kohlendioxid durch den Durchgang über ein Adsorbens erfolgt, welches aus Aktivkohle besteht.

3. Züchtungsverfahren nach Anspruch 2, dadurch gekennzeichnet, daß der Reinigungsdruck in einem Adsorber und der Regenerierungsdruck in einem anderen Adsorber im wesentlichen in der Nachbarschaft des atmosphärischen Druckes liegen.

4. Verfahren nach Anspruch 3, dadurch gekennzeichnet, daß die Dauer der Reinigungsphasen oder der Regenerierungsphasen der adsorber in der Größenordnung von Minuten liegt.

5. Züchtungsverfahren nach Anspruch 4, dadurch gekennzeichnet, daß die Vertauschung der gasförmigen Rezyklierungscharge eines Adsorbers zwecks Reinigung zu einem Adsorber hin zwecks Regenerierung während einer kurzen Zeitdauer erfolgt, indem der Ausgang des Adsorbers am Anfang der Reinigung bei Atmosphäre gehalten wird, derart, daß die Luftbeladung entfernt wird, die er enthält.

6. Züchtungsverfahren von Mikroorganismen nach Anspruch 1, dadurch gekennzeichnet, daß es insbesondere bei den Gärungen mit hoher Zelldichte, insbesondere bei Gärungen mit Ultrafiltration oder Mikrofiltration angewendet wird, wobei die Wachstumshemmer eliminiert werden, mit Zurückhalten und Umwälzen der Mikroorganismen in der Gärvorrichtung.

7. Verfahren nach Anspruch 6, dadurch gekennzeichnet, daß das Substrat in verdünnter Form in Funktion des Stoffwechselbedarfes der Mikroorganismen zugeführt und vollständig von diesen Mikroorganismen verbraucht wird.

8. Züchtungsanlage für aerobe Mikroorganismen in einem Nährsubstrat mit einer Gärvorrichtung mit dichter Abdeckung, die mit einer unteren Leitung mit Öffnungen für die Diffusion eines Sauerstoffanreicherungsgases und einer Meßsonde für den Gehalt an gelöstem Sauerstoff, einer Versorgungsleitung für Sauerstoffanreicherungsgas, welche in der Leitung mit Öffnungen mündet, und einer Entleerungsleitung ausgestattet ist für die Ausstromungen aus der Gärvorrichtung, welche in einer Rezyklierschleife mündet, welche einen Adsorber und einen Kompressor aufweist, gekennzeichnet durch die folgenden Maßnahmen:

a) Die Entleerungsleitung für die Ausströmungen weist aufstromig von der Rezyklierschleife eine Abzweigung auf mit Ventil zu Atmosphäre hin;

b) die Anlage weist einen Satz von zwei Adsorbern auf, deren Eingänge und Ausgänge durch Ventile mit dem Rezyklierkreis einerseits und andererseits durch andere Ventile mit der Atmosphäre und mit einer Umwälzeinrichtung verbunden sind;

c) das Adsorbens ist eine Aktivkohle;

d) die Meßsonde für den Gehalt an gelöstem Sauerstoff wirkt auf einen Vertauschungsschaltkreis, welcher einerseits Vertauschungsventile mit einem Luftkompressor von der Versorgungsleitung her und mit einem Ventil zum Andieluftbringen abstromig von der Gärvorrichtung, und andererseits mindestens ein Ventil zur Verbindung einer Quelle für industriell reinen Sauerstoff sowie ein Rezyklierventil aufweist, sowie auf einen Steuermechanismus für das Ingangsetzen oder Anhalten des Rezyklierkompressors ein.

9. Züchtungsanlage für Mikroorganismen nach Anspruch 8, dadurch gekennzeichnet, daß sie einen Satz von zwei Adsorbern aufweist, deren Eingänge und Ausgänge durch Ventile mit dem Rezyklierkreis einerseits und andererseits durch andere Ventile mit der Atmosphäre und mit einer Umwälzeinrichtung verbunden sind.

10. Züchtungsanlage für Mikroorganismen nach Anspruch 9, dadurch gekennzeichnet, daß sie ein Mittel zum periodischen Vertauschen zweier Adsorber aufweist, um nacheinander eine Reinigungsphase für das ausströmende Gas für den einen und eine Regenerationsphase durch Luft für den anderen und umgekehrt sicherzustellen.

11. Züchtungsanlage für Mikroorganismen nach Anspruch 8, dadurch gekennzeichnet, daß die Gärvorrichtung einer Ultrafiltrations- oder Mikrofiltrationsvorrichtung durch Abzugs- und Rezyklierleitungen für das Kulturmilieu zugeordnet ist, welche an den Enden eines Abteils aufstromig von einer Ultrafiltrations- oder Mikrofiltrationszelle münden, deren abstromiges Abteil mit einer Auslaßleitung mit geregeltem Ventil verbunden ist.

12. Züchtungsanlage für Mikroorganismen nach Anspruch 11, dadurch gekennzeichnet, daß das Regelventil für den Durchsatz an Filtrat durch eine Niveausonde für das Kulturmilieu in der Gärvorrichtung geregelt ist.

13. Anlage nach Anspruch 11, dadurch gekennzeichnet, daß die Gärvorrichtung mit einer Versorgungsleitung für Nährsubstrat mit einer Pumpe für den Durchsatz verbunden ist, die durch das Signal eines Meßgebers geregelt ist, der an der Gärungseinheit oder an den Leitungen für die aus der Gärvorrichtung kommenden, ausströmenden Fließmitteln angebracht ist.

14. Anlage nach Anspruch 11, dadurch gekennzeichnet, daß der Meßgeber eine Sonde für gelösten Sauerstoff oder eine pH-Sonde oder eine Sonde für das Messen der Substratkonzentration ist.

**Claims**

1. Process for the culture of microorganisms under aerobic conditions in a liquid nutritive medium subjected to an oxygenation maintaining the contents of dissolved oxygen in the said medium above a critical freshhold, characterized by the following steps:
a) an initial phase of the culture is assured exclusively by aeration with the atmospheric air, while a phase following this is ensured exclusively by circulation recycling a gaseous charge initially constituted essentially of air, to which industrially clean oxygen is supplied in a regulated manner, which oxygen is determined to compensate substantially the said consumption;
b) the commutation of the initial phase with oxygenation exclusively by air with the subsequent phase with oxygenation by adding exclusively industrially clean oxygen is effected, when the contents of dissolved oxygen is approaching the critical value specific for the cultivated microorganisms.

2. Process for the culture according to claim 1, characterized in that the cleaning of the recycled gaseous charge of carbon dioxide is effected by the passage through an adsorbent consisting of activated carbon.

3. Process for the culture according to claim 2, characterized in that the cleaning pressure in an adsorber and the regeneration pressure in another adsorber are essentially in the neighbourhood of the atmospheric pressure.

4. Process according to claim 3, characterized in that the duration of the cleaning phases or regeneration phases of the adsorbers are in the order of the minute.

5. Process for the culture according to claim 4, characterized in that the commutation of the gaseous recycling charge of an adsorber for the cleaning towards an adsorber for the regeneration is effected during a short period of time maintaining the exit of the said adsorber at atmosphere at the beginning of the cleaning in order to eliminate the charge of air which it comprises.

6. Process for the culture of microorganisms according to claim 1, characterized in that it is applied more particularly to the fermentations with high cellular density especially to fermentations with ultrafiltration or microfiltration permitting to eliminate the growing inhibitors, with retaining and recycling the microorganisms in the fermentation device.

7. Process according to claim 6, characterized in that the substrate is supplied in diluted form in function of the metabolic needs of the microorganisms and integrally consumed by these microorganisms.

8. Installation for the culture of aerobic microorganisms in a nutritive substrate of the type comprising a fermentation device having a tight cover equipped by a lower conduit with openings for the diffusion of an oxygenation gas and a measuring probe for the contents of dissolved oxygen, a feeding conduit for oxygenation gas terminating in the said conduit with openings and of an discharging conduit for the effluences from the fermentation device terminating in a recycling loop incorporating an adsorber and a compressor, characterized by the following measures:
a) the discharging conduit for the effluences, upstream of the said recycling loop comprises a derivation with valve to the atmosphere;
b) the installation comprises a set of two absorbers, the entries and exits of which are connected by valves to the recycling circuit on the one side and on the other side by other valves to the atmosphere and to a circulator;
c) the adsorbent is an activated carbon;
d) the probe for measuring the contents of dissolved oxygen acts on a circuit for commutation comprising on the one side valves communicating to an air compressor from the feeding conduit and downstream of the fermentation device to a valve bringing to the air, and on the other side at least a valve for connecting to a source of industrially clean oxygen and a recycling valve, as well as acting on a mechanism commanding the starting or stopping of a recycling compressor.

9. Installation for the culture of microorganisms according to claim 8, characterized in that it comprises a set of two adsorbers, the entries and exits of which are connected to the recycling circuit on the one side by valves and on the other side by other valves to the atmosphere and to a circulator.

10. Installation for the culture of microorganisms according to claim 9, characterized in that it comprises means for periodically commutating two adsorbers in order to ensure successively a cleaning phase of the effluent gas for the one and a regeneration phase by air for the other and vice-versa.

11. Installation for the culture of microorganisms according to claim 8, characterized in that the fermentation device is associated to an ultrafiltration or microfiltration device by discharging and recycling conduits for the medium of the culture, terminating to the extremities of a compartment upstream of an ultrafiltration or microfiltration cell, the downstream compartment of which being connected to a discharge conduit with regulated valve.

12. Installation for the culture of microorganisms according to claim 11, characterized in that the regulating valve for the contents of the filtrate is controlled by a probe for the level of the medium of the culture in the fermentation device.

13. Installation according to claim 11, characterized in that the fermentation device is connected to a feeding conduit for nutritive substrate incorporating a pump for the throughput controlled by a signal of a sensor installed on the fermentation unit or on the canalisazions of the effluent fluids coming from the fermentation device.

14. Installation according to claim 11, characterized in that the sensor is a probe for dissolved oxygen or a probe pH or a probe for measuring the concentration of the substrate.

FIG.1

0 175 607

FIG.2

0 175 607